# EUROPEAN PATENT SPECIFICATION

(11) **EP 2 966 167 B1**
(45) Date of publication and mention of the grant of the patent: **29.10.2025**
(21) Application number: 14760422.7
(22) Date of filing: 04.03.2014
(51) Int. Cl.: C12N 15/09, A01H 1/00, A01H 5/00, A01H 5/08, C12Q 1/68, C12N 15/82, A01H 1/02, A01H 1/04

(54) **PRSV-RESISTANT CUCUMBER, AND METHOD FOR PRODUCING CUCUMBER EXHIBITING RESISTANCE TO PRSV AND ZYMV**
PRSV-BESTÄNDIGE GURKE UND VERFAHREN ZUR HERSTELLUNG EINER GURKE MIT BESTÄNDIGKEIT GEGEN PRSV UND ZYMV
CONCOMBRE RÉSISTANT AU PRSV, ET PROCÉDÉ DE PRODUCTION DE CONCOMBRE PRÉSENTANT UNE RÉSISTANCE AU PRSV ET AU ZYMV

(30) Priority: 04.03.2013 JP 2013042365
(43) Date of publication of application: 13.01.2016
(73) Proprietor: Takii & Company Limited, Kyoto 600-8686 (JP)
(72) Inventor: HORII, Kiyoshi, Kyoto-shi Kyoto 600-8686 (JP); SHIN, Hisanori, Kyoto-shi Kyoto 600-8686 (JP); ARIMOTO, Ryohei, Kyoto-shi Kyoto 600-8686 (JP); MAEDA, Daisuke, Kyoto-shi Kyoto 600-8686 (JP)
(74) Representative: J A Kemp LLP
(86) International application number: PCT/JP2014/055458
(87) International publication number: WO 2014/136768

(56) References cited:
- WO-A2-2010/020941
- US-A1- 2004 055 033
- US-A1- 2004 055 034
- US-A1- 2012 031 670
- PARK Y H ET AL: "A genetic map of cucumber composed of RAPDs, RFLPs, AFLPs, and loci conditioning resistance to papaya ringspot and zucchini yellow mosaic viruses", GENOME, NATIONAL RESEARCH COUNCIL CANADA, OTTAWA; CA, vol. 43, no. 6, 1 December 2000 (2000-12-01), pages 1003 - 1010, XP002525006, ISSN: 0831-2796, DOI: 10.1139/GEN-43-6-1003
- ADAM D CALL ET AL: "Gene List 2010 for Cucumber Previous Gene Lists", CUCURBIT GENETICS COOPERATIVE REPORT @BULLET WEHNER AND STAUB @BULLET XIE AND WEHNER, 1 January 2010 (2010-01-01), pages 69 - 103, XP055301040, Retrieved from the Internet <URL:http://cuke.hort.ncsu.edu/cgc/cgc3334/cgc3334-23.pdf> [retrieved on 20160908]
- YOUNG HOON Y. ET AL.: "A genetic map of cucumber composed of RAPDs, RFLPs, AFLPs, and loci conditioning resistance to papaya ringspot and zucchini yellow mosaic viruses.", GENOME, vol. 43, no. 6, 2000, pages 1003 - 1010, XP002525006
- CAVATORTA, J. ET AL.: "Marketmore 97': A monoecious slicing cucumber inbred with multiple disease and insect resistances.", HORTSCIENCE, vol. 42, no. 3, 1 June 2007 (2007-06-01), pages 707 - 709, XP055283653
- WAI T. ET AL.: "Inheritance of Resistance to the Watermelon Strain of Papaya ringspot Virus in the Cucumber Line TMG-1.", HORTSCIENCE, vol. 30, no. 2, 1 April 1995 (1995-04-01), pages 338 - 340, XP055283657
- WAI T. ET AL.: "Linkage analysis of potyvirus resistance alleles in cucumber.", JOURNAL OF HEREDITY, vol. 88, no. 6, 1 January 1997 (1997-01-01), pages 454 - 458, XP055283659, DOI: 10.1093/OXFORDJOURNALS.JHERED.A023136
- ZHANG H-Y. ET AL.: "Mapping of 3 major virus resistant genes in cucumber.", NONGYE SHENGWU JISHU XUEBAO, vol. 13, no. 6, 2005, pages 709 - 712, XP008181061

## Description

### Technical Field

The present disclosure relates to PRSV-resistant cucumber plants and a production method of PRSV-resistant and ZYMV-resistant cucumber plants.

### Background Art

In the cultivation of cucumber plants, diseases due to viruses are serious problems on a worldwide basis. In particular, the damages due to the zucchini yellow mosaic virus (ZYMV) and the papaya ringspot virus (PRSV), which are the potyviruses, are enormous.

Specifically, the ZYMV and the papaya ringspot virus type W (PRSV-W) cause acute wilting when cucumber plants are infected with them (Non-Patent Documents 1 and 2). Since virus-infected cucumber plants would die, the damage is enormous. It is said that the economic loss of the damage due to both the viruses in cucumber plant production areas all over Japan is at least about five billion yen a year. In general, the problem is that pharmaceutical drugs effective against these plant viruses have not been reported. Thus, as alternatives, it is necessary to eliminate aphids, which are virus vectors, and to inoculate seedlings with attenuated viruses, and this causes a great deal of labor and cost for the prevention. (Patent Document 1 and Non-Patent Documents 3 and 4).

For solving these problems, the search of virus-resistant cucumber plant lines and the analysis of virus-resistant genes are in progress.

At present, for example, TMG-1 and Dina-1 are known as ZYMV-resistant cucumber plant lines, and TMG-1, Dina-1, and Surinam are known as PRSV-W-resistant cucumber plant lines. There is a report that the TMG-1 and the Dina-1 have the ZYMV resistance controlled by one recessive gene (Non-Patent Document 5). It is said that the ZYMV-resistant gene of the TMG-1 and the ZYMV-resistant gene of the Dina-1 are present in the same gene locus or in gene loci very close to each other (Non-Patent Document 6). It is known that the ZYMV-resistant gene of a cucumber plant is present within 2.2 cM between two markers of the 6^{th} chromosome (Non-Patent Document 7). Also it is known that the PRSV-resistant gene and the ZYMV-resistant gene of the TMG-1 are present close to each other (2.2 cM) and one AFLP marker (E15/M47-F-197) is present at the position 5.2 cM from the ZYMV-resistant gene (Non-Patent Document 8).

In Japan, ZYMV-resistant cucumber plant cultivars are earnestly developed and they are subjected to the cultivation. However, cultivars having the resistance sufficient to both the viruses have not been reported. Since the damage due to both the viruses increases particularly in the hot season, there is a high possibility that the damage increases by the influence of global warming. Thus, there is a need to develop cultivars having the resistance sufficient to not only one of the viruses but both of the viruses.

There is an idea of utilizing a gene recombination technique as a method for giving the resistance. However, consumers still feel insecure about the safety of genetically modified foods. Thus, the provision of cultivars having the resistance to both the viruses without using a gene recombination technique is highly required.

### Citation List

### Patent Document (s)

Patent Document 1: JP 2004-283164 A

### Non-Patent Document(s)

Non-Patent Document 1: "Types of viruses detected from summer-autumn cucumbers produced in Ehime prefecture", Bulletin of the Ehime Prefecture Agricultural Experiment Station/Production Environment Office, 1996
Non-Patent Document 2: Masato IWASAKI, et al. "Type W of Papaya Ringspot Virus (PRSV-W) Isolated from the Wilted Cucumber Plants Grafted on Squash Rootstocks", Japanese Journal of Phytopathology, 1992, p.619
Non-Patent Document 3: You BJ et al., "Engineered Mild Strains of Papaya ringspot virus for Broader Cross Protection in Cucurbits" Phytopathology. 2005 May; 95(5): 533-40
Non-Patent Document 4: Toshihiko SHOJI, "Occurrence of Cucumber Acute Wilting and the Prevention thereof", Plant Protection, 2002, Vol. 56, No. 5, pp.194-197
Non-Patent Document 5: Grumet R et al., "Characterization of sources of resistance to the watermelon strain of Papaya ringspot virus in cucumber: allelism and co-segregation with other potyvirus resistances" Theoretical and Applied Genetics. 2000; 101(3): 463-472
Non-Patent Document 6: Kabelka E et al., "Multiple alleles for zucchini yellow mosaic virus resistance at the zym locus in cucumber" Theoretical and Applied Genetics. 1997, 95(5): 997-1104
Non-Patent Document 7: Yoshiyuki IMURA, "Mapping of zucchini yellow mosaic virus-resistant gene of cucumber", Japanese Journal of Phytopathology, 2011: pp. 239-240
Non-Patent Document 8: Park YH et al., "A genetic map of cucumber composed of RAPDs, RFLPs, AFLPs, and loci conditioning resistance to papaya ringspot and zucchini yellow mosaic viruses" Genome. 2000 Dec; 43(6): 1003-1010

### Brief Summary of the Invention

The invention provides a PRSV-resistance marker for identifying a PRSV-resistance locus on the 6^{th} chromosome of a cucumber plant, which marker is selected from:
(A1) a base sequence of SEQ ID NO: 1;
(A2) a base sequence in which the 25^{th} A in the base sequence of SEQ ID NO: 1 is conserved, having at least 90% identity to the base sequence; and
(A3) a base sequence in which the 25^{th} Ain the base sequence of SEQ ID NO: 1 is conserved and one to twenty bases in the base sequence are deleted, substituted, inserted and/or added.

The invention further provides a use of the marker as defined above for identifying said PRSV-resistance locus.

Yet further provided by the invention is a ZYMV-resistance marker for identifying a ZYMV-resistance locus on the 6^{th} chromosome of a cucumber plant, which marker is selected from:
(B1) a base sequence of SEQ ID NO: 2;
(B2) a base sequence in which the 93^{rd} A and the 307^{th} A in the base sequence of SEQ ID NO: 2 are conserved, having at least 90% identity to the base sequence;
(B3) a base sequence in which the 93^{rd} A and the 307^{th} A in the base sequence of SEQ ID NO: 2 are conserved and one to twenty bases in the base sequence are deleted, substituted, inserted and/or added;
(C1) a base sequence of SEQ ID NO: 3;
(C2) a base sequence in which the 93^{rd} A in the base sequence of SEQ ID NO: 3 is conserved, having at least 90% identity to the base sequence; and
(C3) a base sequence in which the 93^{rd} A in the base sequence of SEQ ID NO: 3 is conserved and one to twenty bases in the base sequence are deleted, substituted, inserted and/or added.

The invention further provides a of the marker as defined above for identifying said ZYMV-resistance locus.

Yet further provided by the invention is a method of selecting a PRSV-resistant and ZYMV-resistant cucumber plant comprising the PRSV-resistance locus from the 6th chromosome of a cucumber plant specified by accession number: FERM BP-11523 and the ZYMV-resistance locus from the 6th chromosome of a cucumber plant specified by accession number: FERM BP-11523; wherein the PRSV-resistance locus and ZYMV-resistance locus are identified using molecular markers as defined above.

The invention further provides a PRSV-resistant and ZYMV-resistant cucumber plant comprising:
(I) a PRSV-resistant gene locus on the 6^{th} chromosome, wherein the PRSV-resistant gene locus is present in a cucumber plant specified by accession number: FERM BP-11523 and defined by a molecular marker selected from the following base sequence (A1), (A2), or (A3):
   (A1) a base sequence of SEQ ID NO: 1;
   (A2) a base sequence in which the 25^{th} A in the base sequence of SEQ ID NO: 1 is conserved, having at least 90% identity to the base sequence, and showing a PRSV resistance; and
   (A3) a base sequence in which the 25^{th} A in the base sequence of SEQ ID NO: 1 is conserved and one to twenty bases in the base sequence are deleted, substituted, inserted and/or added, showing the PRSV resistance; and
(II) a ZYMV-resistant gene locus on the 6^{th} chromosome, wherein the ZYMV-resistant gene locus is defined by a molecular marker selected from the following base sequence (B1), (B2), or (B3):
   (B1) a base sequence of SEQ ID NO: 2;
   (B2) a base sequence in which the 93^{rd} A and the 307^{th} A in the base sequence of SEQ ID NO: 2 are conserved, having at least 90% identity to the base sequence, and showing a ZYMV resistance; and
   (B3) a base sequence in which the 93^{rd} A and the 307^{th} A in the base sequence of SEQ ID NO: 2 are conserved and one to twenty bases in the base sequence are deleted, substituted, inserted and/or added, showing the ZYMV resistance;
   or from the following base sequence (C1), (C2), or (C3):
   (C1) a base sequence of SEQ ID NO: 3;
   (C2) a base sequence in which the 93^{rd} A in the base sequence of SEQ ID NO: 3 is conserved, having at least 90% identity to the base sequence, and showing a ZYMV resistance; and
   (C3) a base sequence in which the 93^{rd} A in the base sequence of SEQ ID NO: 3 is conserved and one to twenty bases in the base sequence are deleted, substituted, inserted and/or added, showing the ZYMV resistance, wherein the PRSV-resistant gene locus and the ZYMV-resistant gene locus are heterozygous.

Further aspects and preferred embodiments are defined in the dependent claims. Any aspects, embodiments and examples of the present disclosure which do not fall under the scope of the appended claims do not form part of the invention and are merely provided for illustrative purposes.

### Brief Summary of Technical Teachings

### Problem to be Solved by the Disclosure

Hence, the present disclosure is intended to provide a new PRSV-resistant cucumber plant and a production method of a PRSV-resistant and ZYMV-resistant cucumber plant using the same.

### Means for Solving Problem

In order to achieve the above object, the present disclosure provides a PRSV-resistant cucumber plant including a PRSV-resistant gene locus on the 6^{th} chromosome, wherein the PRSV-resistant gene locus includes the following base sequence (A1), (A2), or (A3):
(A1) a base sequence of SEQ ID NO: 1;
(A2) a base sequence in which the 25^{th} A in the base sequence of SEQ ID NO: 1 is conserved, having at least 80% identity to the base sequence, and showing a PRSV resistance; and
(A3) a base sequence in which the 25^{th} A in the base sequence of SEQ ID NO: 1 is conserved and one or more bases in the base sequence are deleted, substituted, inserted and/or added, showing the PRSV resistance.

The present disclosure also provides a method of producing a PRSV-resistant and ZYMV-resistant cucumber plant including the following steps (a) and (b):
(a) a step of hybridizing the PRSV-resistant cucumber plant of the present disclosure and a ZYMV-resistant cucumber plant; and
(b) a step of selecting a PRSV-resistant and ZYMV-resistant cucumber plant from the cucumber plants obtained in the step (a) or their progeny lines.

### Effects of the Disclosure

The invention is defined in the appended claims. Any aspects, embodiments and examples of the present disclosure which do not fall under the scope of the appended claims do not form part of the invention and are merely provided for illustrative purposes.

According to the PRSV-resistant cucumber plant disclosed herein, not to mention the PRSV resistance, by hybridizing with a ZYMV-resistant cucumber plant, a cucumber plant having a superior resistance to both the PRSV and the ZYMV can be obtained. Accordingly, it is possible to greatly reduce the yield loss due to the infection with the PRSV and the ZYMV and the labor and cost accompanied with the elimination of vectors and to provide a cucumber plant that satisfies customer needs.

### Brief Description of Drawings

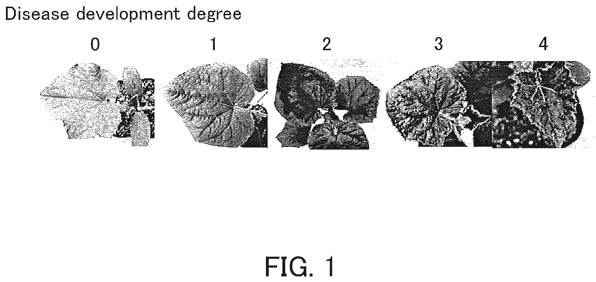

[FIG. 1] FIG. 1 shows photographs for the evaluation criteria of the degree of development of the PRSV in Examples.

### Detailed Description

### (1) PRSV-resistant cucumber plant

As described above, the PRSV-resistant cucumber plant disclosed herein is characterized by including a PRSV-resistant gene locus on the 6^{th} chromosome, wherein the PRSV-resistant gene locus includes the following base sequence (A1), (A2), or (A3):
(A1) a base sequence of SEQ ID NO: 1;
(A2) a base sequence in which the 25^{th} A in the base sequence of SEQ ID NO: 1 is conserved, having at least 80% identity to the base sequence, and showing a PRSV resistance; and
(A3) a base sequence in which the 25^{th} A in the base sequence of SEQ ID NO: 1 is conserved and one or more bases in the base sequence are deleted, substituted, inserted and/or added, showing the PRSV resistance.

In the present disclosure, the "cucumber plant" refers to Cucumis sativus L..

The cultivars of the cucumber plant are not particularly limited, and, for example, can be any type such as Japanese burpless type (Tankei Shiroibo), Suyo Long Cucumber (Yotsuba), English cucumber (Eikoku Onshitsu), Slice cucumber, or Beit-Alpha cucumber. Among them, Japanese burpless type (Tankei Shiroibo) is preferable.

The PRSV-resistant cucumber plant disclosed herein shows the resistance to the PRSV. The PRSV is not particularly limited and examples thereof include PRSV-W (Type W) and PRSV-P (Type P).

In the present disclosure, for example, the "PRSV resistance" is also referred to as the "PRSV tolerance". The resistance means the inhibitory potency or the suppression ability to the occurrence and progression of diseases due to the PRSV infection, for example. Specifically, for example, the resistance may be any of the following: the prevention of the occurrence of diseases, the stop of the progression of occurred diseases, and the suppression (or inhibition) of the progression of occurred diseases. The prevention of the occurrence of diseases and the stop and suppression of the progression of diseases may be achieved by the suppression of the PRSV infection to cells, the suppression of the PRSV growth in infected cells, or the suppression of the PRSV infection (or PRSV spread) from the infected cells to other cells, for example.

In the present disclosure, the PRSV resistance is given by the PRSV-resistant gene locus on the 6^{th} chromosome.

The PRSV-resistant gene locus refers to a quantitative trait locus or a gene region that gives the PRSV resistance. The quantitative trait locus (QTL) generally refers to a chromosome region that involves the expression of quantitative traits. The QTL can be defined by using a molecular marker that shows a specific locus on the chromosome. The technique to define the QTL by using the molecular marker is well known in the art.

In the present disclosure, as described above, the PRSV-resistant gene locus includes the base sequence (A1), (A2), or (A3). In the present disclosure, the PRSV-resistant gene locus may include any of the base sequences (A1), (A2), and (A3).

The base sequence (A1) (SEQ ID NO: 1) is shown in the following table. In the following base sequence, boxed uppercase bases (the 25^{th}, 80^{th}, 118^{th}, 130^{th}, and 317^{th} bases) are single nucleotide polymorphisms (SNPs) that involve the PRSV resistance.

**[Table 1]**

| |
|---|
| SEQ ID NO: 1 |
| agcaatttca aggagcaagc tattAaatac gtgaagcagg cggtacatga agataatgct 60 ggcaattacg caaaagcctT tccgctgtat atgaacgcct tggagtactt caaaactTat 120 ttgaagtatC agaaaaatcc caagatcaag gaagctatta cccagaaatt caccgagtat 180 ttgcgccggg ctgaggagat acgtgctgtt ttggatgatg gcggtcctgg tccggcttcc 240 aatggagatg cagctgttgc aactaaaccc aagacgaaac ctaaggatgg agaaggagga 300 gatggagagg atccagGaca ggctaagtta cgggctggtc ttaattccgc aatcataagg 360 gagaaaccgg atgttaagtg gaacgatgtt gctggattgg agag 404 |

The base sequence (A2) is a base sequence in which the 25^{th} A (SNP) in the base sequence of SEQ ID NO: 1 is conserved, having at least 80% identity to the base sequence, and showing a PRSV resistance. In the base sequence (A2), preferably, the 25^{th} base among the boxed five bases is conserved. Also, in the base sequence (A2), for example, the 25^{th} A may be conserved and at least one of other four bases may be conserved. Preferably, all of the five bases are conserved, although any one of, any two of, any three of, or any four of them may be conserved, for example. With respect to the "identity", it is acceptable as long as the base sequence (A2) is within the range of achieving the PRSV resistance. The identity is preferably at least 85%, more preferably at least 90%, still more preferably at least 95%, still more preferably at least 96%, still more preferably at least 97%, still more preferably at least 98%, and particularly preferably at least 99%. The identity can be determined by the alignment with the base sequence (A1). Specifically, for example, the identity can be calculated by a default parameter by using analysis software such as BLAST, and FASTA (hereinafter, the same applies).

The base sequence (A3) is a base sequence in which the 25^{th} A in the base sequence of SEQ ID NO: 1 is conserved and one or more bases in the base sequence are deleted, substituted, inserted and/or added, showing the PRSV resistance. In the base sequence (A3), preferably, the 25^{th} base among the boxed five bases is conserved. Also, in the base sequence (A3), for example, the 25^{th} A may be conserved and at least one of other four bases may be conserved. Preferably, all of the five bases are conserved, although any one of, any two of, any three of, or any four of them may be conserved, for example. The "one or more" can be any number as long as the base sequence (A3) is within the range of achieving the PRSV resistance. The number of bases to be deleted, substituted, inserted and/or added is, for example, 1 to 20, preferably 1 to 10, more preferably 1 to 9, still more preferably 1 to 5, particularly preferably 1, 2, or 3, and most preferably 1 or 2.

There is no particular limitation on the method of detecting the base sequence (A1), (A2), or (A3). Examples of the method include a method of amplifying a target region by a nucleic acid amplification method such as PCR and analyzing the sequence of the obtained amplification product and analysis methods such as restriction fragment length polymorphism (RFLP). The RFLP can be conducted, for example, as follows. For example, first, the base sequence of the genomic DNA of a cucumber plant of interest is amplified. Next, the obtained amplification product is treated with a restriction enzyme and its fragment pattern is examined. Since the fragment pattern differs between the PRSV-resistant cucumber plant and the PRSV-susceptible cucumber plant depending on the aforementioned SNPs, it is possible to determine the presence or absence of the base sequence based on the fragment pattern. On the basis of the presence or absence of the above-described base sequence, it is possible to determine whether or not the cucumber plant of interest is a PRSV-resistant cucumber plant. The enzyme to be used for the restriction enzyme treatment is not particularly limited and an example thereof includes Mse I. There is no particular limitation on the method of examining the fragment pattern, and an example thereof includes electrophoresis.

The degree of the PRSV resistance can be expressed by the degree of development of the disease according to the method described in Non-Patent Document 5 (Grumet R et al., "Characterization of sources of resistance to the watermelon strain of Papaya ringspot virus in cucumber: allelism and co-segregation with other potyvirus resistances" Theoretical and Applied Genetics. 2000; 101(3):463-472), for example. The description of Example 1 that will be described below can be applied to the calculation of the degree of development of the disease according to this method. For example, the degree of development of the disease may be set as follows: the degree of development of the disease at 1 or less represents the disease resistance and the degree of development of the disease at 3 or more represents the susceptibility.

Examples of the PRSV-resistant cucumber plant disclosed herein include the cucumber plant deposited with the accession number: FERM BP-11523 or its progeny lines. The information of the deposit is as described below.
Type of deposit: International deposit
Name of depository institution: National Institute of Technology and
Evaluation; NITE-IPOD
Accession number: FERM BP-11523
Identifying designation: Takii2
Date of acceptance: December 27, 2012

With respect to the PRSV-resistant cucumber plant disclosed herein, characteristics except for the disease resistance are not particularly limited. For example, morphological characteristics, and biological characteristics are not particularly limited. As a specific example, in the case of a Tankei Shiroibo (short white wart) cucumber, it has dark green cylindrical fruits and it allows easy cultivation management as it has relatively small leaves and forms many offshoots as for its grass shape, for example.

The PRSV-resistant cucumber plant disclosed herein may further have the ZYMV resistance. In this case, the PRSV-resistant cucumber plant disclosed herein may also be referred to as the "PRSV-resistant/ZYMV-resistant cucumber plant" as it has the resistance to both the diseases. The PRSV-resistant/ZYMV-resistant cucumber plant prevents the damages due to both the diseases and thus has a high commercial value. As will be described below, the PRSV-resistant/ZYMV-resistant cucumber plant can be obtained by hybridizing the PRSV-resistant cucumber plant and a ZYMV-resistant cucumber plant. The production method will be described below in detail.

According to the PRSV-resistant cucumber plant disclosed herein, as described above, by hybridizing with a ZYMV-resistant cucumber plant as a parent, it is possible to obtain an F1 cucumber plant having both the PRSV resistance and the ZYMV resistance. The PRSV-resistant cucumber plant disclosed herein to be used as a parent can be obtained by selecting a cucumber plant having the base sequence (A1), (A2), or (A3) or selecting an individual having the PRSV resistance after an inoculation of the PRSV, for example. Among them, the PRSV-resistant cucumber plant to be used as a parent is preferably the cucumber plant identified by the aforementioned accession number or its progeny lines.

In the present disclosure, for example, the "ZYMV resistance" is also referred to as the "ZYMV tolerance". The resistance means the inhibitory potency or the suppression ability to the occurrence and progression of diseases due to the ZYMV infection, for example. Specifically, for example, the resistance may be any of the following: the prevention of the occurrence of diseases, the stop of the progression of occurred diseases, and the suppression (or inhibition) of the progression of occurred diseases. The prevention of the occurrence of diseases and the stop and suppression of the progression of diseases may be achieved by the suppression of the ZYMV infection to cells, the suppression of the ZYMV growth in infected cells, or the suppression of the ZYMV infection (or ZYMV spread) from the infected cells to other cells, for example.

In the case where the PRSV-resistant cucumber plant disclosed herein further has the ZYMV resistance, the ZYMV resistance is given by a ZYMV-resistant gene locus. In the present disclosure, the ZYMV-resistant gene locus is preferably on the 6^{th} chromosome, for example.

The ZYMV-resistant gene locus refers to a quantitative trait locus or a gene region that gives the ZYMV resistance.

Examples of the ZYMV-resistant gene locus include a gene locus (hereinafter, referred to as type I) having the base sequence (B1), (B2), or (B3) and a gene locus (hereinafter, referred to as type II) having the base sequence (C1), (C2), or (C3). In the PRSV-resistant plant disclosed herein, the ZYMV-resistant gene is not particularly limited and may be either the type I or the type II.

(B1) a base sequence of SEQ ID NO: 2;
(B2) a base sequence in which the 93^{rd} A and the 307^{th} Ain the base sequence of SEQ ID NO: 2 are conserved, having at least 80% identity to the base sequence, and showing a ZYMV resistance; and
(B3) a base sequence in which the 93^{rd} A and the 307^{th} Ain the base sequence of SEQ ID NO: 2 are conserved and one or more bases in the base sequence are deleted, substituted, inserted and/or added, showing the ZYMV resistance.

(C1) a base sequence of SEQ ID NO: 3;
(C2) a base sequence in which the 93^{rd} Ain the base sequence of SEQ ID NO: 3 is conserved, having at least 80% identity to the base sequence, and showing a ZYMV resistance; and
(C3) a base sequence in which the 93^{rd} Ain the base sequence of SEQ ID NO: 3 is conserved and one or more bases in the base sequence are deleted, substituted, inserted and/or added, showing the ZYMV resistance.

The base sequence (B1) (SEQ ID NO: 2) is shown in the following table. In the following base sequence, boxed uppercase bases (the 93^{rd} and 307^{th} bases) are SNPs that involve the ZYMV resistance.

**[Table 2]**

| |
|---|
| SEQ ID NO: 2 |
| agcaatttca aggagcaagc tattgaatac gtgaagcagg cggtacatga agataatgct 60 ggcaattacg caaaagcctc tccgctgtat atAaacgcct tggagtactt caaaactcat 120 ttgaagtatg agaaaaatcc caagatcaag gaagctatta cccagaaatt caccgagtat 180 ttgcgccggg ctgaggagat acgtgctgtt ttggatgatg gcggtcctgg tccggcttcc 240 aatggagatg cagctgttgc aactaaaccc aagacgaaac ctaaggatgg agaaggagga 300 gatggaAagg atccagaaca ggctaagtta cgggctggtc ttaattccgc aatcataagg 360 gagaaaccgg atgttaagtg gaacgatgtt gctggattgg agag 404 |

The base sequence (B2) is a base sequence in which the 93^{rd} A and the 307^{th} A in the base sequence of SEQ ID NO: 2 are conserved, having at least 80% identity to the base sequence, and showing a ZYMV resistance. Preferably, all of the two bases are conserved. With respect to the "identity", it is acceptable as long as the base sequence (B2) is within the range of achieving the ZYMV resistance. The identity is preferably at least 85%, more preferably at least 90%, still more preferably at least 95%, still more preferably at least 96%, still more preferably at least 97%, still more preferably at least 98%, and particularly preferably at least 99%. The identity can be determined by the alignment with the base sequence (B1).

The base sequence (B3) is a base sequence in which the 93^{rd} A and the 307^{th} A in the base sequence of SEQ ID NO: 2 are conserved and one or more bases in the base sequence are deleted, substituted, inserted and/or added, showing the ZYMV resistance. Preferably, all of the two bases are conserved. The "one or more" can be any number as long as the base sequence (B3) is within the range of achieving the ZYMV resistance. The number of bases to be deleted, substituted, inserted and/or added is, for example, 1 to 20, preferably 1 to 10, more preferably 1 to 9, still more preferably 1 to 5, particularly preferably 1, 2, or 3, and most preferably 1 or 2.

The base sequence (C1) (SEQ ID NO: 3) is shown in the following table. In the following base sequence, boxed uppercase base (the 93^{rd} base) is a SNP that involves the ZYMV resistance.

**[Table 3]**

| |
|---|
| SEQ ID NO: 3 |
| agcaatttca aggagcaagc tattgaatac gtgaagcagg cggtacatga agataatgct 60 ggcaattacg caaaagcctc tccgctgtat atAaacgcct tggagtactt caaaactcat 120 ttgaagtatg agaaaaatcc caagatcaag gaagctatta cccagaaatt caccgagtat 180 ttgcgccggg ctgaggagat acgtgctgtt ttggatgatg gcggtcctgg tccggcttcc 240 aatggagatg cagctgttgc aactaaaccc aagacgaaac ctaaggatgg agaaggagga 300 gatggagagg atccagaaca ggctaagtta cgggctggtc ttaattccgc aatcataagg 360 gagaaaccgg atgttaagtg gaacgatgtt gctggattgg agag 404 |

The base sequence (C2) is a base sequence in which the 93^{rd} A (SNP) in the base sequence of SEQ ID NO: 3 is conserved, having at least 80% identity to the base sequence, and showing a ZYMV resistance. With respect to the "identity", it is acceptable as long as the base sequence (C2) is within the range of achieving the ZYMV resistance. The identity is preferably at least 85%, more preferably at least 90%, still more preferably at least 95%, still more preferably at least 96%, still more preferably at least 97%, still more preferably at least 98%, and particularly preferably at least 99%. The identity can be determined by the alignment with the base sequence (C1).

The base sequence (C3) is a base sequence in which the 93^{rd} Ain the base sequence of SEQ ID NO: 3 is conserved and one or more bases in the base sequence are deleted, substituted, inserted and/or added, showing the ZYMV resistance. The "one or more" can be any number as long as the base sequence (C3) is within the range of achieving the ZYMV resistance. The number of bases to be deleted, substituted, inserted and/or added is, for example, 1 to 20, preferably 1 to 10, more preferably 1 to 9, still more preferably 1 to 5, particularly preferably 1, 2, or 3, and most preferably 1 or 2.

There is no particular limitation on the methods of detecting the type I base sequence (B1), (B2), or (B3) and the type II base sequence (C1), (C2), or (C3). The detection can be performed in the same manner as the detection method of the base sequence of the PRSV-resistant gene locus described above. As a specific example, the RFLP can be conducted, for example, as follows. For example, first, the type I or type II base sequence of the genomic DNA of a cucumber plant of interest is amplified. Next, the obtained amplification product is treated with a restriction enzyme and its fragment pattern is examined. Since the fragment pattern differs between the ZYMV-resistant cucumber plant and the ZYMV-susceptible cucumber plant depending on the aforementioned SNPs, it is possible to determine the presence or absence of the type I or type II base sequence based on the fragment pattern. On the basis of the presence or absence of the base sequence, it is possible to determine whether or not the cucumber plant of interest is a ZYMV-resistant cucumber plant. Since the fragment pattern differs between the type I base sequence and the type II base sequence, it is possible to classify the base sequences into the type I and the type II. The enzyme to be used for the restriction enzyme treatment is not particularly limited and an example thereof includes Mnl I. There is no particular limitation on the method of examining the fragment pattern, and, as described above, an example thereof includes electrophoresis.

In the PRSV-resistant cucumber plant disclosed herein, the PRSV-resistant gene locus and the ZYMV-resistant gene locus are preferably heterozygotes.

The degree of the ZYMV resistance can be expressed by the degree of development of the disease according to the method described in Non-Patent Document 5 (Grumet R et al., "Characterization of sources of resistance to the watermelon strain of Papaya ringspot virus in cucumber: allelism and co-segregation with other potyvirus resistances" Theoretical and Applied Genetics. 2000; 101(3):463-472), for example. The description of Example 2 that will be described below can be applied to the calculation of the degree of development of the disease according to this method. For example, the degree of development of the disease may be set as follows: the degree of development of the disease at 1 or less represents the disease resistance and the degree of development of the disease at 3 or more represents the susceptibility.

Besides, the degree of the ZYMV resistance can be evaluated by the evaluation criteria for the resistance of a plant at the cultivar registration (http://www.hinsyu.maff.go.jp/info/sinsakijun/botanical_taxon.html#C p35), for example.

In the present disclosure, the "plant body" may refer to either a plant individual representing the whole plant or a part of the plant individual. Examples of the part of the plant individual include organs, tissues, cells, and propagules. The part of the plant individual can be any of them. Examples of the organs include petals, corollas, flowers, leaves, seeds, fruits, stems, and roots. The tissue is a part of the organ, for example. The part of the plant body may be one kind of organs, tissues, and/or cells, or two or more kinds of organs, tissues, and/or cells.

### (2) Production method of PRSV-resistant/ZYMV-resistant cucumber plant

Next, the production method of the PRSV-resistant and ZYMV-resistant cucumber plant (PRSV-resistant/ZYMV-resistant cucumber plant) disclosed herein is described. It is to be noted that the following methods are merely illustrative. In the present disclosure, the production method can be referred to as, for example, a raising method.

As described above, the production method of the PRSV-resistant /ZYMV-resistant cucumber plant disclosed herein is characterized by including the following steps (a) and (b):
(a) a step of hybridizing the PRSV-resistant cucumber plant disclosed herein and a ZYMV-resistant cucumber plant; and
(b) a step of selecting a PRSV-resistant and ZYMV-resistant cucumber plant from the cucumber plants obtained in the step (a) or their progeny lines.

The production method disclosed herein is characterized by using the PRSV-resistant cucumber plant disclosed herein as a parent, and other steps and conditions are not particularly limited.

In the step (a), a PRSV-resistant cucumber plant to be used as the first parent is acceptable as long as it is the PRSV-resistant cucumber plant disclosed herein. The PRSV-resistant cucumber plant is preferably the cucumber plant that is deposited with the accession number: FERM BP-11523 or its progeny line as described above, for example. The PRSV-resistant cucumber plant can be prepared by selecting from test cucumber plants (also referred to as candidate cucumber plants) by the following step (x) prior to the step (a), for example:
(x) a step of selecting the PRSV-resistant cucumber plant disclosed herein.

The selection of the PRSV-resistant cucumber plant in the step (x) can be performed by the following steps (x1) and (x2), for example:
(x1) a step of detecting the presence or absence of the PRSV-resistant gene locus on the chromosome of a test cucumber plan; and
(x2) a step of selecting a test cucumber plant having the PRSV-resistant gene locus as a PRSV-resistant cucumber plant.

The selection of the PRSV-resistant cucumber plant in the step (x) can be performed on the basis of the presence or absence of the base sequence of the PRSV gene locus as described above, for example. That is, in the step (x1), for example, the presence or absence of the PRSV-resistant gene locus is detected on the basis of the presence or absence of the aforementioned base sequence. Thereby, in the step (x2), a cucumber plant having the base sequence can be selected from the test cucumber plants as the PRSV-resistant cucumber plant.

The chromosome in which the presence or absence of the PRSV-resistant gene locus is detected is the 6^{th} chromosome.

In the step (a), a cucumber plant to be used as the other parent is not particularly limited and is acceptable as long as it has the ZYMV resistance. As the ZYMV-resistant cucumber plant to be used for hybridization, for example, a well known ZYMV-resistant cucumber plant may be used or a ZYMV-resistant cucumber plant selected on the basis of whether there is the natural onset of disease or selected after an inoculation of pathogens may be used. There is no particular limitation on the selection method. As a specific example, a ZYMV-resistant cucumber plant having the ZYMV-resistant gene locus on the 6^{th} chromosome can be used, for example. There is no particular limitation on the selection method of the ZYMV-resistant cucumber plant. For example, the ZYMV-resistant cucumber plant can be selected on the basis of the presence or absence of the type I base sequence or the type II base sequence.

Examples of well known ZYMV-resistant cucumber plant include V Road, V Arch, and Slice (TAKII & CO.,LTD.); Kyutaro and Green Sky (Kurume Vegetable Breeding Co., Ltd.); Taibou I and Taibou II (TOKIWA CUCUMBER); Houei V10, Houmi 1, and Houmi 2 (Saitama Gensyu Ikuseikai co,.ltd.); TMG-1; and Dina-1.

In the step (a), there is no particular limitation on the method of hybridizing the PRSV-resistant cucumber plant and the ZYMV-resistant cucumber plant, and well known methods can be employed.

In the step (b), cucumber plants, from which a PRSV-resistant and ZYMV-resistant cucumber plant is selected, may be the cucumber plants obtained in the step (a) or their progeny lines, for example. Specifically, for example, the cucumber plants, from which a PRSV-resistant and ZYMV-resistant cucumber plant is selected, may be the F1 cucumber plants obtained by the hybridization in the step (a) or their progeny lines. The progeny line may be an inbred progeny or a backcross progeny of the F1 cucumber plant obtained by the hybridization in the step (a) or a cucumber plant obtained by hybridizing the F1 cucumber plant and another cucumber plant, for example.

In the step (b), the selection of a PRSV-resistant/ZYMV-resistant cucumber plant can be performed, for example, by directly or indirectly examining the PRSV resistance and the ZYMV resistance.

In the step (b), for example, the direct examination can be performed by making an evaluation on the PRSV resistance and ZYMV resistance of the obtained F1 cucumber plant or its progeny line according to the degree of development of the disease (resistance score) as described above. Specifically, for example, the direct examination can be performed by inoculating the F1 cucumber plant or its progeny line with the PRSV and the ZYMV and making an evaluation on the resistance to the respective diseases according to the degree of development of the disease (resistance score). In this case, for example, an F1 cucumber plant or its progeny line showing the degree of development of the PRSV (PRSV resistance score) at 1 or less and showing the degree of development of the ZYMV (ZYMV resistance score) at 1 or less can be selected as a PRSV-resistant/ZYMV-resistant cucumber plant.

In the step (b), the selection by the indirect examination can be performed by the following steps (b1) and (b2), for example:
(b1) a step of detecting the presence or absence of a PRSV-resistant gene locus and a ZYMV-resistant gene locus on the chromosome of the cucumber plant obtained in the step (a) or its progeny line; and
(b2) a step of selecting the cucumber plant obtained in the step (a) or its progeny line having the PRSV-resistant gene locus and the ZYMV-resistant gene locus as a PRSV-resistant and ZYMV-resistant cucumber plant.

The selection of the PRSV-resistant cucumber plant in the step (b) can be performed by detecting the presence or absence of the PRSV-resistant gene locus in the same manner as described in the step (x), for example. The chromosome in which the presence or absence of the PRSV-resistant gene locus is detected is preferably the 6^{th} chromosome.

In the production method disclosed herein, it is preferable to further raise the PRSV-resistant/ZYMV-resistant cucumber plant selected in the step (b).

In this manner, the cucumber plant or its progeny line which is confirmed to be PRSV-resistant and ZYMV-resistant can be selected as the PRSV-resistant/ZYMV-resistant cucumber plant.

The PRSV-resistant/ZYMV-resistant cucumber plant preferably includes the PRSV-resistant gene locus and the ZYMV-resistant gene locus in the form of heterozygotes, for example.

### Examples

Hereinafter, the present disclosure will be described in detail with reference to examples.

### [Example 1]

With respect to a new PRSV-resistant cucumber plant, the analysis of the PRSV-resistant gene locus was performed.

As the PRSV-resistant cucumber plant, the cucumber plant deposited with the accession number: FERM BP-11523 was used. Hereinafter, this PRSV-resistant cucumber plant is referred to as a deposit line.

An F2 segregating population (inbred progeny) of 104 individuals was obtained by hybridizing the deposit line cucumber plants and PRSV-susceptible line cucumber plants "Hokushin (TAKII & CO.,LTD.)". Then, with respect to 104 individuals of the F2 segregating population, the PRSV inoculation test was performed as follows.

The PRSV inoculation test was performed according to the method described in Non-Patent Document 5. As an inoculum, a filtrate obtained by the following manner was used. The filtrate was obtained by adding ice-cooled 0.02 mol/L sodium phosphate buffer solution (pH7.0) to a young leaf of a PRSV-infected cucumber collected in the field in Miyazaki prefecture in an amount of 10 times (volume), grinding the resultant, and filtrating it with gauze. Then, pots for seedling having a diameter of 9 cm were filled with commercially available seedling soil (Takii seedling soil, TAKII & CO., LTD.) and seeded with the F2 seeds. The soil temperature was kept at 28°C to let the seeds sprout. Seedlings with the cotyledons completely developed were subjected to the PRSV inoculation. The inoculation was performed by lightly sprinkling carborundum on the cotyledons and then applying the inoculum thereto with a cotton swab. After the inoculation, the seedlings were maintained in a greenhouse under the following conditions: the highest temperature at 35°C, the lowest temperature at 25°C, and day length of 13 hours. The degree of development of the disease was examined 14 days after the inoculation.

The resistance was determined with the degree of development of the disease according to the method described in Non-Patent Document 5. FIG. 1 shows photographs of leaves corresponding to the respective degrees of development of the disease.
Degree of development of disease 0
Resistance: no symptom
Degree of development of disease 1
Resistance: minor vein clearing is observed in lower leaves
Degree of development of disease 2
Moderate resistance: although no symptom is observed in young leaves and in the vicinities of growing points, clear leaf curling, mosaic symptom, and vein clearing are observed in lower leaves
Degree of development of disease 3
Susceptibility: leaf curling and vein clearing are observed in the whole plant body
Degree of development of disease 4
Susceptibility: profound leaf curling and vein clearing are observed in the whole plant body

In the F2 segregating population, as a result of the PRSV inoculation, the number of individuals showing the PRSV resistance with the degree of development of the disease at 1 or less was 25 and the number of individuals showing the susceptibility with the degree of development of the disease at 3 or more was 79. There was no individual that shows the moderate resistance. That is, the ratio between the number of PRSV-resistant individuals and the number of PRSV- susceptible individuals was about 1:3. The PRSV-susceptible individuals were taken as PRSV-susceptible lines.

Next, an F3 segregating population (inbred progeny) was obtained from the PRSV-susceptible line individuals. With respect to 10 individuals of the F3 segregating population per line of the PRSV-susceptible line, the PRSV inoculation test was performed in the same manner as described above. As a result, 10 individuals of the F3 segregating population of each line were all PRSV-susceptible or the ratio between the PRSV-resistant individuals (with the degree of development of the disease at 1 or less) and the PRSV-susceptible individuals (with the degree of development of the disease at 3 or more) was about 1:3. It is to be noted that the F3 segregating population (inbred progeny) obtained from the PRSV-resistant line individuals was all PRSV-resistant (with the degree of development of the disease at 1 or less). These results showed that the inheritance of the PRSV-resistant trait is monogenic recessive.

### [Example 2]

With respect to a well known ZYMV-resistant cucumber plant, the analysis of the ZYMV-resistant gene locus was performed.

First, 50 individuals of the inbred progeny (F2 segregating population) of each of F1 "Shakitto (TAKII & CO.,LTD.)" and F1 "V Road (TAKII & CO.,LTD.)", which are known ZYMV-resistant line cucumber plants, were subjected to the inoculation method described below.

The ZYMV inoculation test was performed according to the method described in Non-Patent Document 5. As an inoculum, a filtrate obtained by the following manner was used. The filtrate was obtained by adding ice-cooled 0.02 mol/L sodium phosphate buffer solution (pH7.0) to a young leaf of a ZYMV-infected cucumber collected in the field in Shiga prefecture in an amount of 10 times (volume), grinding the resultant, and filtrating it with gauze. Then, pots for seedling having a diameter of 9 cm were filled with commercially available seedling soil (Takii seedling soil, TAKII & CO., LTD.) and seeded with the F2 seeds. The soil temperature was kept at 28°C to let the seeds sprout. Seedlings with the cotyledons completely developed were subjected to the ZYMV inoculation. The inoculation was performed by lightly sprinkling carborundum on the cotyledons and then applying the inoculum thereto with a cotton swab. After the inoculation, the seedlings were maintained in a greenhouse under the following conditions: the highest temperature at 35°C, the lowest temperature at 25°C, and day length of 13 hours. The degree of development of the disease was examined 7 days after the inoculation.

Then, 1 individual of Shakitto inbred progeny and 1 individual of V Road inbred progeny were respectively obtained from the individuals that show the ZYMV resistance with the degree of development of the disease at 0 by the inoculation test. Hereinafter, the individual of Shakitto inbred progeny is referred to as "type I ZYMV-resistant individual" and the individual of V Road inbred progeny is referred to as "type II ZYMV-resistant individual".

Next, ZYMV-susceptible line cucumber plants "Hokushin (TAKII & CO.,LTD.)" were respectively hybridized with the type I ZYMV-resistant individuals and the type II ZYMV-resistant individuals to obtain two populations (hereinafter, each referred to as a type I population and a type II population) of an F2 segregating population (inbred progeny) of 100 individuals (i.e., 200 individuals in total). Then, with respect to 200 individuals of the F2 segregating populations, the ZYMV inoculation test was performed again.

In the type I population out of the F2 segregating populations, as a result of the ZYMV inoculation, the number of ZYMV-resistant individuals (with the degree of development of the disease at 0) was 23 and the number of susceptible individuals (with the degree of development of the disease at 4) was 77. In the type II population, as a result of the ZYMV inoculation, the number of ZYMV-resistant individuals (with the degree of development of the disease at 0) was 27 and the number of susceptible individuals (with the degree of development of the disease at 4) was 73. That is, both in the type I population and the type II population, the ratio between the number of ZYMV-resistant individuals and the number of ZYMV-susceptible individuals was about 1:3. The ZYMV-susceptible individuals were taken as ZYMV-susceptible lines.

Next, an F3 segregating population (inbred progeny) was obtained from the ZYMV-susceptible line individuals. With respect to 10 individuals of the F3 segregating population per line of the ZYMV-susceptible line, the ZYMV inoculation test was performed in the same manner as described above. As a result, 10 individuals of the F3 segregating population of each line were all ZYMV-susceptible or the ratio between the ZYMV-resistant individuals (with the degree of development of the disease at 1 or less) and the ZYMV-susceptible individuals (with the degree of development of the disease at 3 or more) was about 1:3. It is to be noted that the F3 segregating population (inbred progeny) obtained from the ZYMV-resistant line individuals was all ZYMV-resistant (with the degree of development of the disease at 1 or less). These results showed that the inheritance of the ZYMV-resistant trait is monogenic recessive both in the type I resistant individuals and the type II resistant individuals.

### [Example 3]

The identification of the PRSV-resistant gene locus and the ZYMV-resistant gene locus was performed.

### (1)PRSV-resistant gene locus

With respect to 5 individuals of the PRSV-resistant line cucumber plant of the F2 segregating population obtained in Example 1 and 5 individuals of the ZYMV-resistant line cucumber plant of the F2 segregating population obtained in Example 2, the sequence analysis was performed to about 200 kbp in the peripheral region of two simple sequence repeat (SSR) markers on the 6^{th} chromosome. As a result, specific SNPs were detected at the 25^{th} (A), the 80^{th} (T), the 118^{th} (T), the 130^{th} (C), and the 317^{th} (G) in the region represented by SEQ ID NO: 1.

104 individuals of the F2 segregating population obtained in Example 1 were amplified by PCR, and the sequences of the obtained amplification products were analyzed. A forward primer consisting of the base sequence of SEQ ID NO: 5 and a reverse primer consisting of the base sequence of SEQ ID NO: 6 were used for the PCR. The PCR program was set as follows. One cycle of treatment at 94°C for 1 minute, at 94°C for 30 seconds, at 58°C for 30 seconds, at 72°C for 1 minute, and at 72°C for 5 minutes was repeated 30 cycles.
Forward primer (SEQ ID NO: 5)
   AGCAATTTCAAGGAGCAAGC
Reverse primer (SEQ ID NO: 6)
   CTCTCCAATCCAGCAACAT

The primer set can amplify the base sequence of SEQ ID NO: 4 corresponding to the full-length of the respective base sequences of SEQ ID NO: 1, SEQ ID NO: 2, and SEQ ID NO: 3. In the following sequence, boxed sites indicate the positions of SNPs to be detected.

**[Table 4]**

| |
|---|
| SEQ ID NO: 4 |
| agcaatttca aggagcaagc tattnaatac gtgaagcagg cggtacatga agataatgct 60 ggcaattacg caaaagcctn tccgctgtat atnaacgcct tggagtactt caaaactnat 120 ttgaagtatn agaaaaatcc caagatcaag gaagctatta cccagaaatt caccgagtat 180 |
| ttgcgccggg ctgaggagat acgtgctgtt ttggatgatg gcggtcctgg tccggcttcc 240 aatggagatg cagctgttgc aactaaaccc aagacgaaac ctaaggatgg agaaggagga 300 gatgganagg atccagnaca ggctaagtta cgggctggtc ttaattccgc aatcataagg 360 gagaaaccgg atgttaagtg gaacgatgtt gctggattgg agag 404 |

With respect to the F2 segregating population, the compatibility between the PRSV-resistant trait and the 25^{th}, the 80^{th}, the 118^{th}, the 130^{th}, and the 317^{th} SNPs in the base sequence of SEQ ID NO: 4 was examined. As a result, the PRSV-resistant individual had the following SNPs in the base sequence of SEQ ID NO: 4: A at the 25^{th}, T at the 80^{th}, T at the 118^{th}, C at the 130^{th}, and G at the 317^{th}. Accordingly, it was verified that the PRSV-resistant individual had the PRSV-resistant gene locus. In other words, the PRSV-resistant individual had the PRSV-resistant gene locus represented by the base sequence of SEQ ID NO: 1. Furthermore, it was found that the PRSV-resistant gene locus, which indicates SNPs, of the PRSV-resistant individual was a homozygous. On the other hand, the PRSV-susceptible individual had the following SNPs in the base sequence of SEQ ID NO: 4: G at the 25^{th}, C at the 80^{th}, C at the 118^{th}, G at the 130^{th}, and A at the 317^{th}. Accordingly, it was verified that the PRSV-susceptible individual did not have the PRSV-resistant gene locus represented by the base sequence of SEQ ID NO: 1 but had a susceptible gene locus. Furthermore, it was found that the PRSV-susceptible gene locus of the PRSV-susceptible individual was a homozygous or a heterozygote. These results showed that a PRSV-resistant line cucumber plant can be selected on the basis of the SNPs at the five sites in the base sequence of SEQ ID NO: 1.

### (2)ZYMV-resistant gene locus

100 individuals of the type I population and 100 individuals of the type II population, which are the F2 segregating populations obtained in Example 2, were amplified by PCR in the same manner as in (1), and the sequences of the obtained amplification products were analyzed.

With respect to the F2 segregating population, the compatibility between the ZYMV-resistant trait and the 93^{rd} and the 307^{th} SNPs in the base sequence of SEQ ID NO: 4 was examined. As a result, the ZYMV-resistant individual of the type I population had the following SNPs in the base sequence of SEQ ID NO: 4: A at the 93^{rd} and A at the 307^{th}. Accordingly, it was verified that the type I ZYMV-resistant individual had the type I ZYMV-resistant gene locus. In other words, the type I ZYMV-resistant individual had the type I ZYMV-resistant gene locus represented by the base sequence of SEQ ID NO: 2. Furthermore, it was found that the ZYMV-resistant gene locus, which indicates SNPs, of the type I ZYMV-resistant individual was a homozygous. The ZYMV-resistant individual of the type II population had the following SNP in the base sequence of SEQ ID NO: 4: A at the 93^{rd}. Accordingly, it was verified that the type II ZYMV-resistant individual had the type II ZYMV-resistant gene locus. In other words, the type II ZYMV-resistant individual had the type II ZYMV-resistant gene locus represented by the base sequence of SEQ ID NO: 3. Furthermore, it was found that the ZYMV-resistant gene locus, which indicates SNP, of the type II ZYMV-resistant individual was a homozygous. On the other hand, the ZYMV-susceptible individual had the following SNPs in the base sequence of SEQ ID NO: 4: G at the 93^{rd} and G at the 307^{th}. Accordingly, it was verified that the ZYMV-susceptible individual did not have the ZYMV-resistant gene locus represented by the base sequence of SEQ ID NO: 2 or 3 but had a susceptible gene locus. Furthermore, it was found that the ZYMV-susceptible gene locus of the ZYMV-susceptible individual was a homozygous or a heterozygote. These results showed that a ZYMV-resistant line cucumber plant can be selected on the basis of the SNPs at the two sites in the base sequence of SEQ ID NO: 2 or the SNP at the one site in the base sequence of SEQ ID NO: 3.

### [Example 4]

A PRSV-resistant and ZYMV-resistant cucumber plant was produced.

The deposit line PRSV-resistant cucumber plant (accession number: FERM BP-11523) shown in Example 1 was hybridized with "Natsusuzumi (TAKII & CO.,LTD.)", which is a cucumber plant superior in agronomic traits such as taste, and grass shape to obtain F1 (first filial generation). Then, by inbreeding the F1, 400 individuals of F2 (inbred progeny) were obtained. With respect to the F2 individuals, the sequences were analyzed in the same manner as in Example 3 to select individuals having the PRSV-resistant gene loci. Then, the selected individuals were planted in the field as PRSV-resistant individuals, and the individuals superior in agronomic traits were visually selected. This procedure was repeated 4 times to obtain PRSV-resistant individuals superior in agronomic traits. These are referred to as line A cucumber plants.

On the other hand, the type I individual (type I ZYMV-resistant line cucumber plant) obtained in Example 2 was hybridized with "Natsubayashi (TAKII & CO.,LTD.)", which is a cucumber plant superior in agronomic traits such as taste, and grass shape to obtain F1 (first filial generation). Then, by inbreeding the F1, 398 individuals of F2 (inbred progeny) were obtained. With respect to the F2 individuals, the sequences were analyzed in the same manner as in Example 3 to select the ZYMV-resistant individuals. Then, the selected individuals were planted in the field as ZYMV-resistant individuals, and the individuals superior in agronomic traits were visually selected. This procedure was repeated 4 times to obtain ZYMV-resistant individuals superior in agronomic traits. These are referred to as line B cucumber plants.

Then, PRSV-resistant line A cucumber plants superior in agronomic traits as the first parents were hybridized with type I ZYMV-resistant line B cucumber plants superior in agronomic traits as the second parents to obtain 12 individuals of F1 (first filial generation) line AB. With respect to 12 individuals of the F1 line AB, the PRSV inoculation test and the ZYMV inoculation test were performed in the same manner as in Examples 1 and 2. For comparison, also with respect to 5 individuals of each of the type I individual, the type II individual, "Surinam (North Carolina State University Cucumber Gene Stock Collection; NCG-086)", which is a reported PRSV-resistant cucumber plant, "TMG1 (North Carolina State University Cucumber Gene Stock Collection; NCG-085)", which is a reported ZYMV-resistant cucumber plant, and "Hokushin (TAKII & CO.,LTD.)", which is a cucumber plant susceptible to both the viruses, the inoculation tests were performed in the same manner.

The results of the inoculation tests are summarized in the following Table 5. It was verified that 12 individuals of the F1 line AB all showed the perfect PRSV resistance (with the degree of development of the disease at 0) and ZYMV resistance (with the degree of development of the disease at 0) and maintained the resistance to both the viruses from the beginning of growing to the latter period of growing. That is, it was found that even though the inheritances of both the PRSV gene locus and the ZYMV gene locus are recessive, 12 individuals having these gene loci in the form of heterozygotes have the perfect PRSV resistance and ZYMV resistance.

**[Table 5]**

| | PRSV development degree | ZYMV development degree |
|---|---|---|
| FERM BP-11523 | 0 | 2 |
| Type I ZYMV-resistant individual | 2 | 0 |
| Type II ZYMV-resistant individual | 3 | 0 |
| Surinam | 1 | 4 |
| TMG1 | 3 | 0 |
| Hokushin | 3 | 4 |
| Line AB | 0 | 0 |
| Line AC | 1 | 0 |
| Surinam × type I individual | 1 | 3 |
| Surinam × type II individual | 2 | 3 |

Next, the experiment was performed in the same manner as described above except that the type II ZYMV-resistant line C cucumber plants superior in agronomic traits obtained in Example 2 were used instead of the type I ZYMV-resistant line cucumber plants.

The results of the inoculation tests are summarized in Table 5. It was verified that 8 individuals of the F1 line AC showed the PRSV resistance (with the degree of development of the disease at 1) and the ZYMV resistance (with the degree of development of the disease at 0). That is, similar to the type I individuals, it was found that 8 individuals having the PRSV-resistant gene locus and the type II ZYMV-resistant gene locus in the form of heterozygotes have the PRSV resistance and ZYMV resistance sufficient for breeding.

For comparison, the experiment was performed in the same manner as described above except that "Surinam" cucumber plants, which are reported PRSV-resistant line cucumber plants, were used instead of PRSV-resistant line A cucumber plants. The results thereof are summarized in Table 5. F1 individuals of "Surinam" and the type I ZYMV-resistant cucumber and F1 individuals of "Surinam" and the type II ZYMV-resistant cucumber showed at least the moderate resistance (with the degree of development of the disease at 1, 2) to the PRSV but showed the susceptibility (with the degree of development of the disease at 3) to the ZYMV This result showed that a cucumber plant having the resistance to both the viruses regardless of having the PRSV-resistant gene locus and the ZYMV-resistant gene locus (type I, type II) in the form of heterozygotes is a cucumber plant having the base sequence of SEQ ID NO: 1 as a PRSV-resistant gene locus (for example, a cucumber plant derived from the accession number: FERM BP-11523).

This application claims priority from Japanese Patent Application No. 2013-042365 filed on March 4, 2013.

As described above, PRSV-resistant and ZYMV- resistant cucumber cultivar can be obtained by hybridizing the PRSV-resistant cucumber plant disclosed herein and a ZYMV-resistant cucumber plant. Thus, according to the PRSV-resistant cucumber plant disclosed herein, it is possible to greatly reduce the economic and labor losses such as the yield loss due to the PRSV and ZYMV infection, and the increase in use of agricultural chemicals.

## Claims

1. A PRSV-resistance marker for identifying a PRSV-resistance locus on the 6^{th} chromosome of a cucumber plant, which marker is selected from:
(A1) a base sequence of SEQ ID NO: 1;
(A2) a base sequence in which the 25^{th} A in the base sequence of SEQ ID NO: 1 is conserved, having at least 90% identity to the base sequence; and
(A3) a base sequence in which the 25^{th} Ain the base sequence of SEQ ID NO: 1 is conserved and one to twenty bases in the base sequence are deleted, substituted, inserted and/or added.

2. Use of the marker of claim 1 for identifying said PRSV-resistance locus.

3. A ZYMV-resistance marker for identifying a ZYMV-resistance locus on the 6^{th} chromosome of a cucumber plant, which marker is selected from:
(B1) a base sequence of SEQ ID NO: 2;
(B2) a base sequence in which the 93^{rd} A and the 307^{th} Ain the base sequence of SEQ ID NO: 2 are conserved, having at least 90% identity to the base sequence;
(B3) a base sequence in which the 93^{rd} A and the 307^{th} Ain the base sequence of SEQ ID NO: 2 are conserved and one to twenty bases in the base sequence are deleted, substituted, inserted and/or added;
(C1) a base sequence of SEQ ID NO: 3;
(C2) a base sequence in which the 93^{rd} Ain the base sequence of SEQ ID NO: 3 is conserved, having at least 90% identity to the base sequence; and
(C3) a base sequence in which the 93^{rd} Ain the base sequence of SEQ ID NO: 3 is conserved and one to twenty bases in the base sequence are deleted, substituted, inserted and/or added.

4. Use of the marker of claim 3 for identifying said ZYMV-resistance locus.

5. A method of selecting a PRSV-resistant and ZYMV-resistant cucumber plant comprising the PRSV-resistance locus from the 6^{th} chromosome of a cucumber plant specified by accession number: FERM BP-11523 and the ZYMV-resistance locus from the 6^{th} chromosome of a cucumber plant specified by accession number: FERM BP-11523; wherein the PRSV-resistance locus and ZYMV-resistance locus are identified using molecular markers as defined in claim 1 and claim 3.

6. A PRSV-resistant and ZYMV-resistant cucumber plant comprising:
(I) a PRSV-resistant gene locus on the 6^{th} chromosome, wherein the PRSV-resistant gene locus is present in a cucumber plant specified by accession number: FERM BP-11523 and defined by a molecular marker selected from the following base sequence (A1), (A2), or (A3):
(A1) a base sequence of SEQ ID NO: 1;
(A2) a base sequence in which the 25^{th} A in the base sequence of SEQ ID NO: 1 is conserved, having at least 90% identity to the base sequence, and showing a PRSV resistance; and
(A3) a base sequence in which the 25^{th} A in the base sequence of SEQ ID NO: 1 is conserved and one to twenty bases in the base sequence are deleted, substituted, inserted and/or added, showing the PRSV resistance; and
(II) a ZYMV-resistant gene locus on the 6^{th} chromosome, wherein the ZYMV-resistant gene locus is defined by a molecular marker selected from the following base sequence (B1), (B2), or (B3):
(B1) a base sequence of SEQ ID NO: 2;
(B2) a base sequence in which the 93^{rd} A and the 307^{th} Ain the base sequence of SEQ ID NO: 2 are conserved, having at least 90% identity to the base sequence, and showing a ZYMV resistance; and
(B3) a base sequence in which the 93^{rd} A and the 307^{th} Ain the base sequence of SEQ ID NO: 2 are conserved and one to twenty bases in the base sequence are deleted, substituted, inserted and/or added, showing the ZYMV resistance;
or from the following base sequence (C1), (C2), or (C3):
(C1) a base sequence of SEQ ID NO: 3;
(C2) a base sequence in which the 93^{rd} Ain the base sequence of SEQ ID NO: 3 is conserved, having at least 90% identity to the base sequence, and showing a ZYMV resistance; and
(C3) a base sequence in which the 93^{rd} Ain the base sequence of SEQ ID NO: 3 is conserved and one to twenty bases in the base sequence are deleted, substituted, inserted and/or added, showing the ZYMV resistance, wherein the PRSV-resistant gene locus and the ZYMV-resistant gene locus are heterozygous.

7. The PRSV-resistant and ZYMV-resistant cucumber plant according to claim 6, wherein the PRSV-resistant and ZYMV-resistant cucumber plant is a plant body or a part of the plant body.

8. The PRSV-resistant and ZYMV-resistant cucumber plant according to claim 6 or 7, wherein the PRSV-resistant and ZYMV-resistant cucumber plant is a seed.

## Patentansprüche

1. APRSV-Resistenzmarker für die Identifizierung eines PRSV-Resistenz-Locus auf dem 6. Chromosom einer Gurkenpflanze, wobei der Marker ausgewählt ist aus:
(A1) einer Basensequenz der SEQ ID NO: 1;
(A2) einer Basensequenz, in der das 25. A in der Basensequenz von SEQ ID NO: 1 konserviert ist, die wenigstens 90 % Identität mit der Basensequenz aufweist; und
(A3) einer Basensequenz, in der der 25. A in der Basensequenz von SEQ ID NO: 1 konserviert ist und eine bis zwanzig Basen in der Basensequenz deletiert, substituiert, eingefügt und/oder hinzugefügt sind.

2. Verwendung des Markers nach Anspruch 1 für die Identifizierung des PRSV-Resistenz-Locus.

3. ZYMV-Resistenzmarker für die Identifizierung eines ZYMV-Resistenz-Locus auf dem 6. Chromosom einer Gurkenpflanze, wobei der Marker ausgewählt ist aus:
(B1) einer Basensequenz der SEQ ID NO: 2;
(B2) einer Basensequenz, in der das 93. A und das 307. A in der Basensequenz von SEQ ID NO: 2 konserviert sind, die wenigstens 90 % Identität mit der Basensequenz aufweist; und
(B3) einer Basensequenz, in der der 93. A und das 307. A in der Basensequenz von SEQ ID NO: 2 konserviert sind und eine bis zwanzig Basen in der Basensequenz deletiert, substituiert, eingefügt und/oder hinzugefügt sind;
(C1) einer Basensequenz von SEQ ID NO: 3;
(C2) einer Basensequenz, in der das 93. A in der Basensequenz von SEQ ID NO: 3 konserviert ist, die wenigstens 90 % Identität mit der Basensequenz aufweist; und
(C3) einer Basensequenz, in der der 93. A in der Basensequenz von SEQ ID NO: 3 konserviert ist und eine bis zwanzig Basen in der Basensequenz deletiert, substituiert, eingefügt und/oder hinzugefügt sind.

4. Verwendung des Markers nach Anspruch 3 für die Identifizierung des ZYMV-Resistenz-Locus.

5. Verfahren zum Auswählen einer PRSV-resistenten und ZYMV-resistenten Gurkenpflanze, umfassend den PRSV-Resistenz-Locus aus dem 6. Chromosom einer Gurkenpflanze, die durch die Hinterlegungsnummer spezifiziert ist: FERM BP-11523, und den ZYMV-Resistenz-Locus aus dem 6. Chromosom einer Gurkenpflanze, die durch die Hinterlegungsnummer spezifiziert ist: FERM BP-11523; wobei der PRSV-Resistenz-Locus und der ZYMV-Resistenz-Locus unter Verwendung molekularer Marker nach Anspruch 1 und Anspruch 3 identifiziert werden.

6. PRSV-resistente und ZYMV-resistente Gurkenpflanze, umfassend:
(I) einen PRSV-resistenten Gen-Locus auf dem 6. Chromosom, wobei der PRSV-resistente Gen-Locus in einer Gurkenpflanze vorhanden ist, die durch die Hinterlegungsnummer: FERM BP-11523 spezifiziert ist und durch einen molekularen Marker definiert ist, der aus den folgenden Basensequenzen (A1), (A2) oder (A3) ausgewählt ist:
(A1) einer Basensequenz der SEQ ID NO: 1;
(A2) einer Basensequenz, in der das 25. A in der Basensequenz von SEQ ID NO: 1 konserviert ist, die wenigstens 90 % Identität mit der Basensequenz aufweist und eine PRSV-Resistenz zeigt; und
(A3) einer Basensequenz, in der der 25. A in der Basensequenz von SEQ ID NO: 1 konserviert ist und eine bis zwanzig Basen in der Basensequenz deletiert, substituiert, eingefügt und/oder hinzugefügt sind, die die PRSV-Resistenz zeigen; und
(II) einen ZYMV-resistenten Gen-Locus auf dem 6. Chromosom, wobei der ZYMV-resistente Gen-Locus durch einen molekularen Marker definiert ist, der aus den folgenden Basensequenzen (B1), (B2) oder (B3) ausgewählt ist:
(B1) einer Basensequenz der SEQ ID NO: 2;
(B2) einer Basensequenz, in der das 93. A und das 307. A in der Basensequenz von SEQ ID NO: 2 konserviert sind, die wenigstens 90 % Identität mit der Basensequenz aufweist und eine ZYMV-Resistenz zeigt; und
(B3) einer Basensequenz, in der der 93. A und das 307. A in der Basensequenz von SEQ ID NO: 2 konserviert sind und eine bis zwanzig Basen in der Basensequenz deletiert, substituiert, eingefügt und/oder hinzugefügt sind, die die ZYMV-Resistenz zeigen;
oder aus den folgenden Basensequenzen (C1), (C2) oder (C3):
(C1) einer Basensequenz von SEQ ID NO: 3;
(C2) einer Basensequenz, in der das 93. A in der Basensequenz von SEQ ID NO: 3 konserviert ist, die wenigstens 90 % Identität mit der Basensequenz aufweist und eine ZYMV-Resistenz zeigt; und
(C3) einer Basensequenz, in der der 93. A in der Basensequenz von SEQ ID NO: 3 konserviert ist und eine bis zwanzig Basen in der Basensequenz deletiert, substituiert, eingefügt und/oder hinzugefügt sind, die die ZYMV-Resistenz zeigen, wobei der PRSV-resistente Gen-Locus und der ZYMV-resistente Gen-Locus heterozygot sind.

7. PRSV-resistente und ZYMV-resistente Gurkenpflanze nach Anspruch 6, wobei die PRSV-resistente und ZYMV-resistente Gurkenpflanze ein Pflanzenkörper oder ein Teil des Pflanzenkörpers ist.

8. PRSV-resistente und ZYMV-resistente Gurkenpflanze nach Anspruch 6 oder 7, wobei die PRSV-resistente und ZYMV-resistente Gurkenpflanze ein Samen ist.

## Revendications

1. Marqueur de résistance au PRSV pour identifier un locus de résistance au PRSV sur le 6^{e} chromosome d'un plant de concombre, lequel marqueur est sélectionné parmi :
(A1) une séquence de base de SEQ ID N° : 1 ;
(A2) une séquence de base dans laquelle le 25^{e} A de la séquence de base de SEQ ID N° : 1 est conservé, présentant au moins 90 % d'identité avec la séquence de base ; et
(A3) une séquence de base dans laquelle le 25^{e} A de la séquence de base de SEQ ID N° : 1 est conservé et une à vingt bases de la séquence de base sont délétées, substituées, insérées et/ou ajoutées.

2. Utilisation du marqueur de la revendication 1 pour identifier ledit locus de résistance au PRSV.

3. Marqueur de résistance au ZYMV pour identifier un locus de résistance au ZYMV sur le 6^{e} chromosome d'un plant de concombre, lequel marqueur est sélectionné parmi :
(B1) une séquence de base de SEQ ID N° : 2 ;
(B2) une séquence de base dans laquelle le 93^{e} A et le 307^{e} A de la séquence de base de SEQ ID N° : 2 sont conservés, présentant au moins 90 % d'identité avec la séquence de base ;
(B3) une séquence de base dans laquelle le 93^{e} A et 307^{e} A de la séquence de base de SEQ ID N° : 2 sont conservés et une à vingt bases de la séquence de base sont délétées, substituées, insérées et/ou ajoutées ;
(C1) une séquence de base de SEQ ID N° : 3 ;
(C2) une séquence de base dans laquelle le 93^{e} A de la séquence de base de SEQ ID N° : 3 est conservé, présentant au moins 90 % d'identité avec la séquence de base ; et
(C3) une séquence de base dans laquelle le 93^{e} A de la séquence de base de SEQ ID N° : 3 est conservé et une à vingt bases de la séquence de base sont délétées, substituées, insérées et/ou ajoutées.

4. Utilisation du marqueur de la revendication 3 pour identifier ledit locus de résistance au ZYMV.

5. Procédé de sélection d'un plant de concombre résistant au PRSV et résistant au ZYMV comprenant le locus de résistance au PRSV provenant du 6^{e} chromosome d'un plant de concombre spécifié par le numéro d'accession : FERM BP-11523 et le locus de résistance au ZYMV provenant du 6^{e} chromosome d'un plant de concombre spécifié par le numéro d'accession : FERM BP-11523 ; dans lequel le locus de résistance au PRSV et le locus de résistance au ZYMV sont identifiés à l'aide de marqueurs moléculaires tels que définis dans la revendication 1 et la revendication 3.

6. Plant de concombre résistant au PRSV et résistant au ZYMV comprenant :
(I) un locus de gène résistant au PRSV sur le 6^{e} chromosome, dans lequel le locus de gène résistant au PRSV est présent dans un plant de concombre spécifié par le numéro d'accession : FERM BP-11523 et défini par un marqueur moléculaire sélectionné parmi les séquences de bases (A1), (A2), ou (A3) suivantes :
(A1) une séquence de base de SEQ ID N° : 1 ;
(A2) une séquence de base dans laquelle le 25^{e} A de la séquence de base de SEQ ID N° : 1 est conservé, présentant au moins 90 % d'identité avec la séquence de base, et montrant une résistance au PRSV ; et
(A3) une séquence de base dans laquelle le 25^{e} A de la séquence de base de SEQ ID N° : 1 est conservé et une à vingt bases de la séquence de base sont délétées, substituées, insérées et/ou ajoutées, montrant la résistance au PRSV ; et
(II) un locus de gène résistant au ZYMV sur le 6^{e} chromosome, dans lequel le locus de gène résistant au ZYMV est défini par un marqueur moléculaire sélectionné parmi les séquences de bases (B1), (B2) ou (B3) suivantes :
(B1) une séquence de base de SEQ ID N° : 2 ;
(B2) une séquence de base dans laquelle le 93^{e} A et le 307^{e} A de la séquence de base de SEQ ID N° : 2 sont conservés, présentant au moins 90 % d'identité avec la séquence de base, et montrant une résistance au ZYMV ; et
(B3) une séquence de base dans laquelle le 93^{e} A et 307^{e} A de la séquence de base de SEQ ID N° : 2 sont conservés et une à vingt bases de la séquence de base sont délétées, substituées, insérées et/ou ajoutées, montrant la résistance au ZYMV ;
ou à partir de la séquence de base (C1), (C2), ou (C3) suivante :
(C1) une séquence de base de SEQ ID N° : 3 ;
(C2) une séquence de base dans laquelle le 93^{e} A de la séquence de base de SEQ ID N° : 3 est conservé, présentant au moins 90 % d'identité avec la séquence de base, et montrant la résistance au ZYMV ; et
(C3) une séquence de base dans laquelle le 93^{e} A de la séquence de base de SEQ ID N° : 3 est conservé et une à vingt bases de la séquence de base sont délétées, substituées, insérées et/ou ajoutées, montrant la résistance au ZYMV, dans lequel le locus de gène résistant au PRSV et le locus de gène résistant au ZYMV sont hétérozygotes.

7. Plant de concombre résistant au PRSV et résistant au ZYMV selon la revendication 6, dans lequel le plant de concombre résistant au PRSV et résistant au ZYMV est un corps de plant ou une partie du corps de plant.

8. Plant de concombre résistant au PRSV et résistant au ZYMV selon la revendication 6 ou 7, dans lequel le plant de concombre résistant au PRSV et résistant au ZYMV est une graine.
